# EUROPÄISCHE PATENTANMELDUNG

(11) **EP 2 082 730 A2**
(43) Veröffentlichungstag der Anmeldung: **29.07.2009**
(21) Anmeldenummer: 09150405.0
(22) Anmeldetag: 12.01.2009
(51) Int. Cl.: A61K 8/96, A61K 8/97, A61Q 19/08

(54) **Kosmetische Zusammensetzung mit Anti-Alterungswirkung und deren Verwendung zur Anti-Faltenbehandlung**

(30) Priorität: 11.01.2008 DE 102008004665
(71) Anmelder: Coty Prestige Lancaster Group GmbH, 55116 Mainz (DE)
(72) Erfinder: Golz-Berner, Karin, 98000, Monaco (MC); Zastrow, Leonhard, 98000, Monaco (MC)
(74) Vertreter: Gulde Hengelhaupt Ziebig & Schneider

(57) **Zusammenfassung**

Die Erfindung betrifft eine kosmetische Zusammensetzung mit verstärkter Anti-Alterungswirkung der Haut und die Verwendung dieser Zusammensetzung zur Anti-Faltenbehandlung.

Die kosmetische Zusammensetzung umfasst ein Gemisch eines Blütenextraktes der blauen Lotusblume Nymphaea cearulea, eines pulverisierten Jade-Minerals und eines Orchideenblütenextraktes, ausgewählt unter den Orchideenblütenextrakten aus Gymnadenia austriaca, Nigritella nigra, Encyclia odoratissima, Maxillaria tenuifolia, sowie kosmetisch annehmbare Hilfsstoffe, Trägerstoffe und Gemische davon.

Die Zusammensetzung zeigt eine sehr schnell auftretende Anti-Faltenwirkung ohne Hautirritationen durch Parfümöle.

## Beschreibung

Die Erfindung betrifft eine kosmetische Zusammensetzung mit verstärkter Anti-Alterungswirkung, insbesondere verbesserter Anti-Faltenwirkung und die Verwendung dieser kosmetischen Zusammensetzung zur Faltenbehandlung.

In der US-B-6468564 ist eine topische Zusammensetzung mit Anti-Faltenwirkung beschrieben, die verschiedene Lotusextrakte enthält und nach 4 Wochen gute Ergebnisse zeigt.

Die EP-B-236374 offenbart die Verwendung von diversen Edelsteinpulvern zu dekorativen Zwecken in kosmetischen Zusammensetzungen.

Die Erfindung stellt sich die Aufgabe, eine zeitlich schnell erreichbare Anti-Alterungswirkung, insbesondere Anti-Faltenwirkung, für die menschliche Haut über ein entsprechendes Kosmetikum bereitzustellen.

Eine weitere Aufgabe ist die gleichzeitige Herabsetzung der Hautirritation, die durch Parfümöle hervorgerufen wird.

Eine weitere Aufgabe der Erfindung ist es, die Anti-Faltenwirkung durch eine feuchtigkeitserhaltende Wirkung zu stützen.

Erfindungsgemäß werden die Aufgaben gelöst durch eine kosmetische Zusammensetzung mit Anti-Alterungswirkung, die ein Gemisch eines Blütenextraktes der blauen Lotusblume Nymphaea cearulea, eines pulverisierten Jade-Minerals und eines Orchideenblütenextraktes, ausgewählt unter den Orchideenblütenextrakten mit den INCI-Namen Gymnadenia Austriaca Flower Extract, Nigritella Nigra Flower Extract, Encyclia Odoratissima Flower Extract, Maxillaria Tenuifolia Flower Extract, umfasst, sowie kosmetisch annehmbare Hilfsstoffe, Trägerstoffe und Gemische davon.

Der Extrakt der blauen Lotusblume Nymphaea cearulea ist ein handelsüblicher Blütenextrakt, der durch Extraktion der blauen Lotusblüten bei Umgebungstemperatur (18 - 25°C) mit Glycerin oder Glycerin/Wasser hergestellt wird (INCI: Nymphaea Cearulea Flower Extract; CAS number: 999 999-99-4).

Jade oder Jadeit ist ein Natrium-Aluminiumsilikat und tritt je nach geringen Fremdmetalleinschlüssen von Chrom oder Mangan in grünen bis gelben, seltener violetten Erscheinungsformen auf. Die Teilchengröße des eingesetzten gemahlenen Jadeits beträgt 1 - 50 µm, bevorzugt 1 - 40 µm, insbesondere 3 - 30 µm. Erfindungsgemäß besonders bevorzugt ist gemahlenes grünes Jade-Mineral.

Die Kombination von blauem Lotusblütenextrakt und gemahlener Jade zeigt eine besonders starke Anti-Alterungswirkung auf der Haut, insbesondere bei kleinen Hautfalten im Gesichtsbereich. Überraschenderweise tritt eine nachweisbare Wirkung sehr schnell auf, innerhalb von 3 - 8 Tagen, und nicht erst nach mehreren Wochen, wie es für den blauen Lotusextrakt allein zu erwarten war. Innerhalb von 3 - 8 Tagen ist bereits eine signifikante Reduzierung der Anzahl der Falten und der Faltentiefe zu verzeichnen. Gegenstand der vorliegenden Erfindung ist deshalb auch die Verwendung der erfindungsgemäßen kosmetischen Zusammensetzung zur Faltenreduzierung, vorzugsweise im Gesichtsbereich, insbesondere in den Augenwinkeln und an der Mundpartie.

Darüber hinaus scheint sich der Verzicht auf den Zusatz von Parfümölen zur Zusammensetzung der Erfindung, die im Normalfall immer ein Gemisch von vielen Ölen darstellen und deren irritative Wirkungen im Gemisch bekannt sind, zusätzlich positiv auf die Beschleunigung der Anti-Alterungswirkung auszuwirken. Die erfindungsgemäßen kosmetischen Zusammensetzungen sind daher besonders für die sensitive Haut geeignet.

Das erfindungsgemäße kosmetische Produkt enthält neben den genannten Wirkstoffen weitere kosmetisch annehmbare Hilfsstoffe oder Trägerstoffe oder Gemische davon. Als kosmetische Hilfsstoffe werden in diesem Zusammenhang alle Begleitstoffe bezeichnet, die gegebenenfalls auch eine eigene Wirkung aufweisen, wie z.B. UV-Filter oder die Bräunung oder Aufhellung der Haut beeinflussende Mittel, Radikalfänger, Enzyme, Wachse, pflanzliche Wirkstoffe, Polymere, Melanin, Antioxidationsmittel, entzündungswidrige natürliche Wirkstoffe, Ubichinon Q10, Kreatin, Kreatinin, Camitin, Biotin, Isoflavone, Isoflavonoide, Cardiolipin, Liponsäure, sowie Hautfunktionen verbessernde Wirkstoffe wie Grüntee-Extrakte, Eucalyptusöl, Harnstoff, Mineralsalze, Meeresmineralien und Osmolyte. Kiwi-Fruchtextrakt und Süssholzextrakt gemäß DE 10 2004 042 299 A1 als kosmetisch annehmbare Hilfs- oder Trägerstoffe sind in der kosmetischen Zusammensetzung der vorliegenden Erfindung nicht enthalten.

Weitere Hilfsstoffe sind Konservierungsmittel, Farbstoffe, Pigmente mit färbender Wirkung, Verdickungsmittel, Alkohole, Polyole, Ester, Elektrolyte, Gelbildner, Polymere, Copolymere, Wachse, Stabilisatoren, Emulgatoren.

Zu Antioxidationsmitteln gehören Vitamine wie Vitamin C und Derivate davon, beispielsweise Ascorbylacetat, -phosphat und -palmitat, Magnesiumascorbylphosphat; Folsäure und deren Derivate; Vitamin E und deren Derivate, wie Tocopherylacetat; Flavone oder Flavonoide; Aminosäuren, wie Histidin, Glycin, Tyrosin, Tryptophan und Derivate davon; Imidazole wie z.B. cis- oder trans-Urocaninsäure und deren Derivate; Peptide wie D,L-Carnosin, D-Carnosin, L-Carnosin und deren Derivate; Carotinoide und Carotine, wie z.B α-Carotin, β-Carotin, Lycopin; Harnsäure und Derivate davon; α-Hydroxysäuren wie Citronensäure, Milchsäure, Apfelsäure; α-Hydroxyfettsäuren wie Palmitinsäure, Phytinsäure, Lactoferrin; Stilbene und deren Derivate; Mannose und deren Derivate; Ferulasäure und deren Derivate; Thiole wie Glutathion, Cystein, Cystin und deren Ester.

Ein bevorzugtes Antioxidationsmittel ist ein Gemisch mit einem Gehalt an einem durch Extraktion der Rinde von Quebracho blanco und nachfolgender enzymatischer Hydrolyse gewonnenem Produkt, das wenigstens 90 Gew- % Proanthocyanidin-Oligomere und höchstens 10 Gew-% Gallussäure enthält, in Mikrokapseln, sowie mit einem durch Extraktion gewonnenen Seidenraupenextrakt, der das Peptid Cecropine, Aminosäuren und ein Vitamingemisch enthält, und einem nichtionischen, kationischen oder anionischen Hydro-Gel oder Gemisch von Hydro-Gelen, und einem oder mehreren Phospholipiden, und Wasser (RPF-Komplex 1 gemäß Anspruch 1 der WO 99/66881).

Ein weiteres bevorzugtes Antioxidationsmittel ist ein Gemisch aus Angelica archangelica-Wurzelextrakt, Pongamia pinnata-Samenextrakt, Camelia sinensis-Blattextrakt, Coffea arabica-Samenextrakt, Ethanol im Verhältnis 0,2 : 0,2 : 0,2 : 0,2 : 99,2 gemäß Anspruch 1 der WO 2004/105706 (RPF-Komplex 2).

Als sehr gut geeignetes Antioxidationsmittel kann auch ein RPF-Komplex (als "RPF-Komplex classic" bezeichnet) eingesetzt werden, der ein in Lecithin verkapseltes Gemisch aus (in Gew.- %) 2 % Angelica archangelica-Wurzelextrakt (INCI: Angelica archangelica root extract, CAS number 84775-41-7), 2 % Pongamia pinnata-Samenextrakt (INCI: Pongamia pinnata seed extract), 2 % Camelia sinensis-Blattextrakt (INCI: Camellia sinensis Leaf Extract, CAS number 84650-60-2), 2 % Coffea arabica-Samenextrakt (INCI: Coffea arabica (coffee) seed extract, CAS number 84650-00-0) beinhaltet, wobei außerdem 8 % Glycerin, 8,25 % Alkohol denaturiert, Antioxidationsmittel und Hilfsstoffe enthalten sind.

Erfindungsgemäß kann die kosmetische Zusammensetzung der Erfindung auch ein Radikalfängergemisch aus dem RPF-Komplex 1 und dem RPF-Komplex 2 oder dem RPF-Komplex 1 und dem RPF-Komplex classic umfassen.

Als Wachse können natürliche pflanzliche Wachse, tierische Wachse, natürliche und synthetische Mineralwachse und synthetische Wachse verwendet werden. Dazu gehören beispielsweise Carnaubawachs, Candellilawachs, Bienenwachs, Wollwachs, Hartparaffin, Ceresin, Ozokerit, Siliconwachs, Polyethylenglycolwachse oder Polyethylenglycolesterwachse.

Als Trägerstoffe können Wasser, polare und unpolare Öle, bestimmte nicht oder wenig färbende Pigmente oder Pulver eingesetzt werden.

Zu Pigmenten für das erfindungsgemäße Produkt gehören Titandioxid, Zinkoxid, Aluminiumoxid, Siliciumdioxid, Silicate, Glimmer, Kaolin, Calciumcarbonat, Talkum, Nylonkügelchen, Keramikkügelchen, expandierte und nichtexpandierte synthetische Polymerpulver, pulverförmige natürliche organische Verbindungen wie gemahlene Festalgen, gemahlene Pflanzenteile, verkapselte und unverkapselte Getreidestärken.

Die für die Erfindung eingesetzten Öle können übliche kosmetische Öle sein oder kosmetische Ester oder Ether. Besonders geeignete kosmetische Öle sind beispielsweise Siliconöle, Mineralöle, Hydrogenated Polyisobuten, Polyisopren, Squalane, Tridecyltrimellitat, Trimethylpropan-triisostearat, Isodecylcitrat, Neopentylglycoldi-heptanoat, PPG-15-stearylether sowie pflanzliche Öle, wie Calendulaöl, Jojobaöl, Avocadoöl, Macadamianussöl, Rizinusöl, Kakaobutter, Kokosnussoil, Maisöl, Baumwollsamenöl, Olivenöl, Palmkernöl, Rapssamenöl, Safloröl, Sesamsamenöl, Sojabohnenöl, Sonnenblumensamenöl, Weizenkeimöl, Traubenkernöl, Kukuinuss-öl, Distelöl und Gemische davon. Parfümöle werden der erfindungsgemäßen Zusammensetzung nicht zugesetzt.

Als Ester oder Ether sind zum Beispiel geeignet Glyceryl Stearate, PEG -100 Stearate, Ceteareth-20, Propylene Glycol Dioctanoate, Propylene Glycol Dicaprylate-2,30-Dicaprate, Tridecyl Stearate/Neopentyl Glycol Dicaprylate, Dicaprate/Tridecyl Trimellitate, Neopentyl Glycol Dioctanoate, Isopropyl Myristate, Diisopropyl Dimer Dilinoleate, Trimethylpropane Triisostearate, Myristyl Ether, Stearyl Ether, Cetearyl Octanoate, Butyl Ether, Dicaprylyl Ether, PPG1-PEG9 Lauroyl Glycol Ether, PPG15 Stearyl Ether, PPG14 Butyl Ether, Fomblin HC25.

Es ist weiterhin vorteilhaft, dem erfindungsgemäßen Produkt entsprechende wasser- und/oder öllösliche UVA- oder UVB-Filter oder beide zuzusetzen. Zu vorteilhaften öllöslichen UVB-Filtern gehören 4-Aminobenzoesäure-Derivate wie der 4-(Dimethylamino)-benzoesäure-(2-ethylhexyl)ester; Ester der Zimtsäure wie der 4-Methoxyzimtsäure(2-ethylhexyl)ester; Benzophenon-Derivate wie 2-Hydroxy-4-methoxybenzophenon; 3-Benzylidencampher-Derivate wie 3-Benzylidencampher.

Bevorzugte öllösliche UV Filter sind Benzophenone-3, Butyl-Methoxybenzoyl-methane, Octyl Methoxycinnamate, Octyl Salicylate, 4-Methylbenzylidene Camphor, Homosalate und Octyl Dimethyl PABA.

Wasserlösliche UVB-Filter sind z.B. Sulfonsäurederivate von Benzophenon oder von 3-Benzylidencampher oder Salze wie das Na- oder K-Salz der 2-Phenylbenzimidazol-5-sulfonsäure.

Zu UVA-Filtern gehören Dibenzoylmethan-Derivate wie 1-Phenyl-4-(4'-isopropylphenyl)propan-1,3-dion, Butyl Methoxybenzoyl-methane oder Menthyl Anthranilate.

Besonders bevorzugt sind Benzophenone-3, Butyl Methoxydibenzoylmethane, Octyl Methoxycinnamate, Octyl Salicylate, 4-Methylbenzylidene Camphor, Homosalate, Octocrylene, Ethylhexyl Methoxycinnamate, Isoamyl-p-Methoxycinnamate, Octyl Dimethyl PABA, Ethylhexyltriazone, Diethylhexyl Butamido Triazone, Ethylhexyl Salicylate, Methylene Bis-Benzotriazolyl Tetramethylbutylphenol, Disodium Phenyl Dibenzimidazole Tetrasulfonate, Bis-Ethylhexyloxyphenol Methoxyphenyl Triazine.

Weiterhin können auch Breitbandfilter eingesetzt werden, wie z.B. Bis-Resorcinyltriazin-Derivate, oder auch Benzoxazole.

Weiterhin einsetzbar als Sonnenschutzfilter sind anorganische Pigmente auf Basis von Metalloxiden, wie TiO₂, SiO₂, ZnO, Fe₂O₃, ZrO₂, MnO, Al₂O₃, die auch im Gemisch verwendet werden können.

Als Erweichungsmittel können normalerweise eine Vielzahl von Verbindungen eingesetzt werden, wie Stearylalkohol, Glycerylmonoricinoleat, Glycerylmonostearat, Propan-1,2-diol, Butan-1,3-diol, Cetylalkohol, Isopropylisostearat, Stearinsäure, Isobutylpalmitat, Isopropylmyristat, Isopropylpalmitat, Oleylalkohol, Isopropyllaurat, Decyloleat, Octadecan-2-ol, Isocetylalkohol, Cetylpalmitat, Siliconöle wie Dimethylpolysiloxan, Polyethylenglycol, Lanolin, Kakaobutter, pflanzliche Öle wie Maisöl, Baumwollsamenöl, Olivenöl, mineralische Öle, Butylmyristat, Palmitinsäure usw. Der Zusatz von Erweichungsmitteln ist jedoch nicht bevorzugt.

Die erfindungsgemäße kosmetische Zusammensetzung kann z.B. als Serum, Creme, Gel, Milch, Lotion, Maske oder Puder verwendet werden. Die Anwendung kann damit z. B. in Form von Sonnencremes, Sonnengelen, After-sun-Produkten, Tagescremes, Nachtcremes, Masken, Körperlotionen, Reinigungsmilch, Körperpuder, Augenkosmetik, Duschgelen, Duschölen, Badeölen und in Produkten der dekorativen Kosmetik wie z. B. Rouge, Grundierung, Make-up erfolgen. Die Anwendung der erfindungsgemäßen kosmetischen Zusammensetzung als Creme oder Serum ist bevorzugt.

Die Herstellung derartiger Produkte erfolgt auf eine Weise, wie sie dem Fachmann auf diesem Gebiet bekannt ist.

Die Erfindung soll nachstehend durch Beispiele näher erläutert werden. Alle Angaben erfolgen in Gewichtsprozent, sofern nichts anderes angegeben ist.

### Beispiel 1 Antifaltencreme I

| **Phase A** | |
|---|---|
| Wasser | q.s. ad 100 |
| Glycerine | 4,0 |
| Propylene Glycole | 2,0 |
| Carbomer | 0,5 |
| Ethylene Diamine Tetra Acetic Acid (EDTA) | 0,1 |

| **Phase B** | |
|---|---|
| Glyceryl Stearate | 2,5 |
| PEG-100 Stearate | 2,0 |
| Ceteareth - 20 | 3,5 |

| **Phase C** | |
|---|---|
| Silicone Oil (Dimethicone) | 7,5 |
| Ethanol | 3,0 |
| Konservierungsmittel | 0,5 |

| **Phase D** | |
|---|---|
| Jadepulver 1 - 50 µm (grün) | 1,0 |
| Encyclia Odoratissima Flower Extract | 2,0 |
| Nymphaea cearulea Flower Extract | 5,0 |

Die Phasen A und B wurden separate bei etwa 80°C hergestellt und dann unter Rühren zusammengeführt. Nach dem Abkühlen auf etwa 40°C wurde die Phase C hinzugegeben. Danach erfolgte die Zugabe der Phase D bei etwa 30°C.

### Beispiel 2 Antifalten-Reinigungscreme für fettige Haut

| **Phase A** | |
|---|---|
| Wasser | q.s. ad 100 |
| Glycerine | 2,5 |
| EDTA | 0,1 |

| **Phase B** | |
|---|---|
| Beheneth-10 | 4,0 |
| Behenyl Alcohol | 1,5 |
| Shea Butter | 3,0 |

| **Phase C** | |
|---|---|
| Xanthan Gum | 0,2 |
| Ethanol | 10,0 |
| RPF-Komplex 2** | 0,5 |

| **Phase D** | |
|---|---|
| Jadepulver (grün) | 0,5 |
| Maxillaria tenuifolia Flower Extract | 2,0 |
| Nymphaea cearulea Flower Extract | 1,0 |

| | |
|---|---|
| **RPF-Komplex 2: Angelica archangelica-Wurzelextrakt, Pongamia pinnata-Samenextrakt, Camelia sinensis-Blattextrakt, Coffea arabica-Samenextrakt, Ethanol im Verhältnis 0,2 : 0,2 : 0,2 : 0,2 : 99,2 gemäß Anspruch 1 der WO 2004/105706. | |

Die Verarbeitung erfolgte wie im Beispiel 1.

### Beispiel 3 Antifalten-Reinigungscreme für fettige Haut

| **Phase A** | |
|---|---|
| Wasser | q.s. ad 100 |
| Glycerine | 2,5 |
| EDTA | 0,1 |

| **Phase B** | |
|---|---|
| Beheneth-10 | 4,0 |
| Behenyl Alcohol | 1,5 |
| Shea Butter | 3,0 |

| **Phase C** | |
|---|---|
| Xanthan Gum | 0,2 |
| Ethanol | 10,0 |
| RPF-Komplex classic** | 0,5 |

| **Phase D** | |
|---|---|
| Jadepulver (grün) | 0,5 |
| Maxillaria tenuifolia Flower Extract | 2,0 |
| Nymphaea cearulea Flower Extract | 1,0 |

| | |
|---|---|
| ** RPF-Komplex classic bestehend aus 3,5 % Lecithin, 2 % Angelica archangelica root extract, 2 % Pongamia pinnata seed extract, 2 % Camelia sinensis Leaf Extract, 2 % Coffea arabica seed extract, 8 % Glycerin, 8,25 % Alkohol denaturiert, 0,3 % Zitronensäure, 0,2 % Ascorbyl Palmitate, 0,2 % Tocopherol, 0,2 % Ascorbinsäure, 1,0 % Guar Hydroxypropyltrimonium chlorid, 0,2 % PEG-8, 0,28 % Konservierungsmittel und q. s. ad 100 Wasser, wobei der RPF-Komplex classic in Lecithin verkapselt vorliegt. | |

### Beispiel 4 Serum

| **Phase A** | |
|---|---|
| Wasser | q.s. ad 100 |
| Glycerine | 5,0 |
| EDTA | 0,1 |

| **Phase B** | |
|---|---|
| Cetearyl Alcohol | 3,0 |
| PEG-100 Stearate | 2,0 |
| Dpraprylyl Carbonate | |
| Tocopherol | |

| **Phase C** | |
|---|---|
| Silicone Oil (Cyclohexasiloxane & Cyclopentasiloxane | |
| & Cyclotetrasiloxane) | 2,0 |
| Konservierungsmittel | 0,5 |
| Ethanol | 1,0 |
| RPF Komplex 1 * | 0,5 |

| **Phase D** | |
|---|---|
| Jadepulver (grün) | 0,05 |
| Nymphaea Cearulea Flower Extract | 2,0 |
| Gymnadenia Austriaca Flower Extract | 3,0. |

| | |
|---|---|
| * RPF-Komplex 1, bestehend aus Wasser, 1 % Gelbildner, 7,5 % Phospholipide, 2 % Quebracho-Extrakt und 1 % Seidenraupen-Extrakt (gemäß Anspruch 1 der WO99/66881), bezogen auf das Gesamtgewicht des Komplexes. | |

Die Verarbeitung erfolgte wie im Beispiel 1.

### Beispiel 5 Serum

| **Phase A** | |
|---|---|
| Wasser | q.s. ad 100 |
| Glycerine | 5,0 |
| EDTA | 0,1 |

| **Phase B** | |
|---|---|
| Cetearyl Alcohol | 3,0 |
| PEG-100 Stearate | 2,0 |
| Dpraprylyl Carbonate | |
| Tocopherol | |

| **Phase C** | |
|---|---|
| Silicone Oil (Cyclohexasiloxane & Cyclopentasiloxane | |
| & Cyclotetrasiloxane) | 2,0 |
| Konservierungsmittel | 0,5 |
| Ethanol | 1,0 |
| RPF Komplex classic ** | 0,5 |

| **Phase D** | |
|---|---|
| Jadepulver (grün) | 0,05 |
| Nymphaea Cearulea Flower Extract | 2,0 |
| Gymnadenia Austriaca Flower Extract | 3,0. |

| | |
|---|---|
| ** RPF-Komplex classic, siehe Beispiel 3 | |

### Beispiel 6 Anwender-Test

Ein Anwendertest mit 16 Teilnehmerinnen im Alter von 38 bis 57 Jahren wurde durchgeführt. 8 Teilnehmerinnen (Gruppe A) applizierten zweimal täglich eine Creme gemäß Beispiel 1 im Gesicht. Die zweite Gruppe (B) applizierte eine Creme gemäß Beispiel 1, jedoch ohne die Bestandteile der Phase D. Dafür wurde der Anteil an Wasser erhöht.

In den Abständen 1 Tag, 3 Tage, 5 Tage, 8 Tage wurden digitalisierte Aufnahmen gemacht, insbesondere vom Augenwinkel und der Mundpartie der Teilnehmerinnen, und über ein Computerprogramm nach Anzahl der Falten und Faltentiefe ausgewertet.

Die Ergebnisse sind in Tabelle 1 dargestellt.

**Tabelle 1**

| **Faltenreduzierung [%]** | | |
|---|---|---|
| | **Gruppe A** | **Gruppe B** |
| Behandlungsbeginn | 0 | 0 |
| nach 1 Tag | 4 | 0 |
| nach 3 Tagen | 7-10 | 1-3 |
| nach 5 Tagen | 15-19 | 1-3 |
| nach 8 Tagen | 28-32 | 3-4 |

Tabelle 1 zeigt deutlich die Überlegenheit der Antifaltencreme I mit der erfindungsgemäßen Zusammensetzung (Gruppe A) gegenüber der Anwendung ohne die erfindungsgemäßen Wirkstoffe (Gruppe B).

In einem weiteren Test mit der Zusammensetzung (B) und zusätzlich 5 % Nymphaea cearulea Flower Extract wurde ebenfalls innerhalb von 8 Tagen ein gewisser Anstieg der Antifaltenwirkung beobachtet, der jedoch unter 15 % lag.

## Patentansprüche

1. Kosmetische Zusammensetzung mit Anti-Alterungswirkung, **dadurch gekennzeichnet, dass** die Zusammensetzung aus einem Gemisch eines Blütenextraktes der blauen Lotusblume Nymphaea cearulea, eines pulverisierten Jade-Minerals und eines Orchideenblütenextraktes, ausgewählt aus der Gruppe der Orchideenblütenextrakte aus Gymnadenia austriaca, Nigritella nigra, Encyclia odoratissima, Maxillaria tenuifolia und kosmetisch annehmbaren Hilfsstoffen, Trägerstoffen oder Gemischen davon besteht.

2. Zusammensetzung nach Anspruch 1, **dadurch gekennzeichnet, dass** der Anteil des Lotusblumenextraktes im Bereich von 0,1 - 5 Gew.-% liegt, bezogen auf das Gewicht der Zusammensetzung.

3. Zusammensetzung nach Anspruch 1, **dadurch gekennzeichnet, dass** der Anteil des Jade-Minerals im Bereich von 0,01 - 3 Gew.-% liegt, bezogen auf das Gewicht der Zusammensetzung.

4. Zusammensetzung nach Anspruch 1, **dadurch gekennzeichnet, dass** der Anteil des Orchideenextraktes im Bereich von 1,5 - 5 Gew.-% liegt, bezogen auf das Gewicht der Zusammensetzung, insbesondere im Bereich von 1,5 - 3 Gew.-%.

5. Zusammensetzung nach Anspruch 1 - 4, **dadurch gekennzeichnet, dass** sie keine zugesetzten Parfümöle enthält.

6. Zusammensetzung nach Anspruch 1, **dadurch gekennzeichnet, dass** sie als Hilfsstoff einen Radikalfänger oder ein Gemisch von mehreren Radikalfängern enthält.

7. Zusammensetzung nach Anspruch 6, **dadurch gekennzeichnet, dass** das Radikalfänger-Gemisch ein Pflanzenextraktgemisch ist, ausgewählt unter einem Assoziationskomplex, bestehend aus einem Produkt, gewonnen durch Extraktion der Rinde von Quebracho blanco und nachfolgender enzymatischer Hydrolyse, das wenigstens 90 Gew.-% Proanthocyanidin-Oligomere und höchstens 10 Gew.-% Gallussäure enthält, in Mikrokapseln, sowie mit einem durch Extraktion gewonnenen Seidenraupenextrakt, der das Peptid Cecropine, Aminosäuren und ein Vitamingemisch enthält, und einem nicht-ionischen, kationischen oder anionischen Hydro-Gel oder Gemisch von Hydro-Gelen, und einem oder mehreren Phospholipiden, und Wasser;
und einem Gemisch aus Angelica archangelica-Wurzelextrakt, Pongamia pinnata-Samenextrakt, Camelia sinensis-Blattextrakt, Coffea arabica-Samenextrakt, Ethanol im Verhältnis 0,2 : 0,2 : 0,2 : 0,2 : 99,2 oder einem in Lecithin verkapseltem Gemisch aus (in Gew.- %) 2 % Angelica archangelica-Wurzelextrakt, 2 % Pongamia pinnata-Samenextrakt, 2 % Camelia sinensis-Blattextrakt, 2 % Coffea arabica-Samenextrakt, wobei außerdem 8 % Glycerin, 8,25 % Alkohol denaturiert, Antioxidationsmittel und Hilfsstoffe enthalten sind.

8. Verwendung der kosmetischen Zusammensetzung gemäß der Ansprüche 1 bis 7 zur Faltenbehandlung, vorzugsweise im Gesichtsbereich, insbesondere in den Augenwinkeln und an der Mundpartie.

9. Verwendung nach Anspruch 8 zur Faltenbehandlung sensitiver Haut.
